# EUROPEAN PATENT SPECIFICATION

(11) **EP 4 606 307 B1**
(45) Date of publication and mention of the grant of the patent: **18.11.2026**
(21) Application number: 25158946.1
(22) Date of filing: 19.02.2025
(51) Int. Cl.: A61B 6/00

(54) **INFORMATION PROCESSING APPARATUS, MEDICAL IMAGE CAPTURING APPARATUS, INFORMATION PROCESSING METHOD, AND INFORMATION PROCESSING PROGRAM**
INFORMATIONSVERARBEITUNGSVORRICHTUNG, MEDIZINISCHE BILDERFASSUNGSVORRICHTUNG, INFORMATIONSVERARBEITUNGSVERFAHREN UND INFORMATIONSVERARBEITUNGSPROGRAMM
APPAREIL DE TRAITEMENT D'INFORMATIONS, APPAREIL DE CAPTURE D'IMAGE MÉDICALE, PROCÉDÉ DE TRAITEMENT D'INFORMATIONS ET PROGRAMME DE TRAITEMENT D'INFORMATIONS

(30) Priority: 22.02.2024 JP 2024025609
(43) Date of publication of application: 27.08.2025
(73) Proprietor: FUJIFILM Corporation, Tokyo 106-8620 (JP)
(72) Inventor: TAKEMOTO, Kazuma, Tokyo, 106-8620 (JP)
(74) Representative: Dehns Germany Partnerschaft mbB

(56) References cited:
- EP-A1- 4 052 651
- EP-A1- 4 052 652
- US-A1- 2015 327 830
- US-A1- 2021 077 054
- US-A1- 2021 201 476

## Description

### BACKGROUND OF THE INVENTION

### 1. Field of the Invention

The present disclosure relates to an information processing apparatus, a medical image capturing apparatus, an information processing method, and an information processing program.

### 2. Description of the Related Art

JP2014-128656A discloses an automatic scanning and positioning apparatus of a scout image, the apparatus comprising a reception unit that receives a scout image of a subject obtained through scout scanning, an examination and determination unit that automatically examines at least one position in the scout image according to a plurality of pieces of information included in the scout image and automatically determines a scanning range based on the at least one position, and an execution unit that executes axial scanning on the subject based on the determined scanning range. The scout image referred to here is also referred to as a scanogram image, a topogram, and the like, and will be referred to as a "scanogram image" below.

According to this technique, it is possible to reduce the time and effort in a case where an operator manually sets an imaging range (scanning range) and improve an inaccuracy in setting the imaging range. EP 4 052 651 A1 relates to an image-based planning of a tomographic scan.

### SUMMARY OF THE INVENTION

In the technique disclosed in JP2014-128656A, an imaging range for main imaging is automatically set based on features of the scanogram image for positioning. However, for a subject having a relatively thick body thickness, there is a problem in that the imaging range may be insufficient in a case where only the scanogram image from a front direction (anterior-posterior view (AP) direction) is captured, and in an axial image obtained by the main imaging, a thick portion of the body may be chipped.

The present disclosure has been made in view of the above circumstances, and an object of the present disclosure is to provide an information processing apparatus, a medical image capturing apparatus, an information processing method, and an information processing program capable of suppressing occurrence of chipping in an axial image obtained by main imaging.

According to a first aspect of the present disclosure, there is provided an information processing apparatus, method and storage medium as defined in independent claims 1, 7 and 8, respectively. Particular embodiments are defined in the dependent claims.

According to the present disclosure, it is possible to provide an information processing apparatus, a medical image capturing apparatus, an information processing method, and an information processing program capable of suppressing the occurrence of chipping in an axial image obtained by main imaging.

### BRIEF DESCRIPTION OF THE DRAWINGS

Fig. 1 is a diagram showing a schematic configuration of a medical diagnostic apparatus according to an embodiment of the disclosed technology.
Fig. 2 is a block diagram showing an example of a functional configuration of a console according to the embodiment of the disclosed technology.
Fig. 3 is a diagram for describing a problem of the related art, and is a diagram showing an example of an imaging range of main imaging and an axial image obtained by the related art.
Fig. 4 is a diagram for describing a problem of the related art, and is a side view showing an example of a medical image capturing apparatus and a graph showing a relationship between a position on a top plate of an examination table and a body thickness.
Fig. 5 is a diagram for describing the disclosed technology, and is a diagram showing an example of an imaging range of main imaging and an axial image obtained by the disclosed technology.
Fig. 6 is a diagram for describing a first embodiment of the disclosed technology, in which an upper diagram is a diagram showing an example of an imaging range of the main imaging and an axial image obtained in the same manner as the related art by the disclosed technology, and a lower diagram is a diagram showing an example of an imaging range of the main imaging and an axial image obtained by the disclosed technology.
Fig. 7 is a flowchart showing a flow of control of the medical diagnostic apparatus by a console according to the first embodiment of the disclosed technology.
Fig. 8 is a diagram for describing a second embodiment of the disclosed technology, in which an upper diagram is a diagram showing an example of an imaging range of the main imaging and an axial image obtained by the disclosed technology, and a lower diagram is a diagram showing an example of an imaging range of the main imaging and an axial image obtained by adding a margin by the disclosed technology.
Fig. 9 is a diagram for describing a problem of the disclosed technology, and is a diagram showing an example of a defect in an axial image caused by narrowing an imaging range due to misunderstanding by a user.
Fig. 10 is a diagram showing an example of a state of a notification on a graphical user interface (GUI) screen displayed in a case of planning main imaging according to a third embodiment of the disclosed technology.
Fig. 11 is a block diagram showing an example of a functional configuration of a console according to the third embodiment of the disclosed technology.
Fig. 12 is a flowchart showing a flow of control of the medical diagnostic apparatus by the console according to the third embodiment of the disclosed technology.
Fig. 13 is a graph showing an example of changes in respiratory information and body thickness over time according to a fourth embodiment of the disclosed technology.
Fig. 14 is a map showing an example of a relationship between a respiratory timing and a ratio of change in body thickness according to the fourth embodiment of the disclosed technology.
Fig. 15 is a diagram for describing a fifth embodiment of the disclosed technology, in which a left diagram is a diagram showing an example of a defect in an axial image by the disclosed technology, and a right diagram is a diagram showing an example of an axial image in which the defect is eliminated by reconstruction by the disclosed technology.

### DESCRIPTION OF THE PREFERRED EMBODIMENTS

Hereinafter, examples of embodiments of the present disclosure will be described with reference to the accompanying drawings. In each drawing, the same or equivalent constituent elements and parts are designated by the same reference numerals. Dimensional ratios in the drawings are exaggerated for convenience of description and may be different from actual ratios.

### First Embodiment

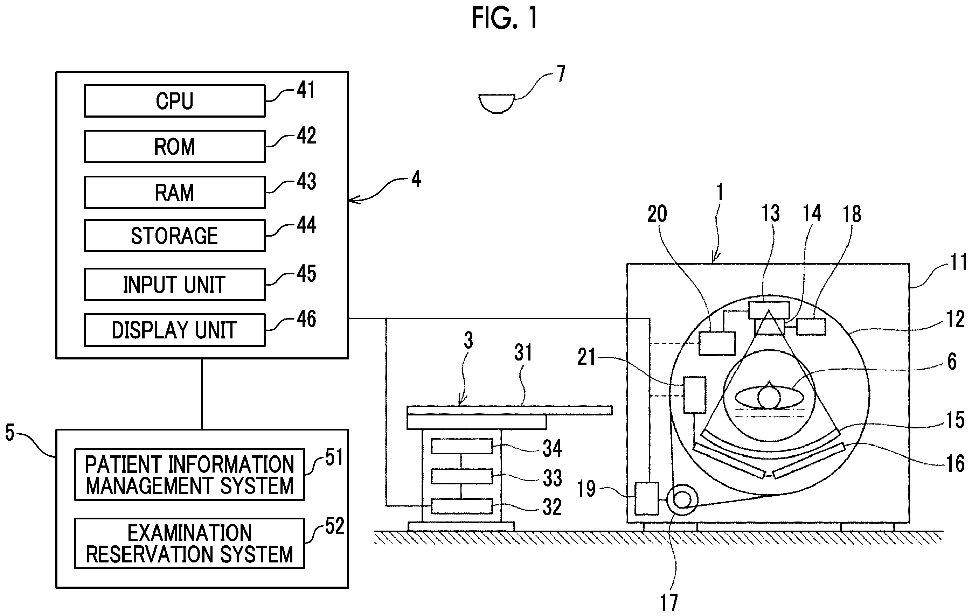

Fig. 1 is a diagram showing a schematic configuration of a medical diagnostic apparatus including an information processing apparatus and a medical image capturing apparatus according to the present embodiment. In the present embodiment, a configuration of an embodiment of an X-ray computed tomography (CT) apparatus as the medical diagnostic apparatus is shown. The medical diagnostic apparatus of the present disclosure is not limited to the X-ray CT apparatus, and can be applied to other medical diagnostic apparatuses such as a magnetic resonance imaging (MRI) apparatus.

The medical diagnostic apparatus according to the present embodiment includes a scanner 1, an examination table 3, and a console 4. The console 4 corresponds to an information processing apparatus according to the present disclosure, the scanner 1 corresponds to a radiography apparatus according to the present disclosure, and a combination of the scanner 1, the examination table 3, and the console 4 corresponds to a medical image capturing apparatus according to the present disclosure.

The scanner 1 is a portion that executes CT scanning. The scanner 1 comprises a gantry 11, a rotating plate 12 that has an opening at a center portion and is rotatably supported by the gantry 11, an X-ray tube device 13 fixed to the rotating plate 12, a collimator 14 provided in an X-ray emission port portion of the X-ray tube device 13, an X-ray detector 15 that is disposed to face the X-ray tube device 13 with the opening of the rotating plate 12 interposed therebetween, and a rotating plate drive unit 17 provided in the gantry 11. In addition, the rotating plate 12 of the scanner 1 comprises a collimator controller 18 that controls the collimator 14 to change an X-ray irradiation field, a rotating plate drive controller 19 that performs drive control of the rotating plate drive unit 17, an X-ray high-voltage generation device 20 that supplies power for generating X-rays to the X-ray tube device 13 and controls X-ray generation conditions, a data collection device 16 that collects the output of the X-ray detector 15, and a data transmission device 21 that transmits the data collected by the data collection device 16. The power and control signals are supplied to each unit provided in the rotating plate 12, and the data is taken out from each unit provided in the rotating plate 12, via a slip ring (not shown) provided between the gantry 11 and the rotating plate 12.

The examination table 3 moves a subject between an imaging preparation position and an imaging position. The subject is placed on a top plate 31. Although not shown, an up-down movement mechanism and a front-rear movement mechanism of the top plate 31 are provided. In addition, the examination table 3 is provided with an examination table controller 32, a top plate up-down movement controller 33, and a top plate front-rear movement controller 34 for controlling operations of the up-down movement mechanism and the front-rear movement mechanism of the top plate 31.

The console 4 controls the medical diagnostic apparatus that is the X-ray CT apparatus. The console 4 is an example of a computer according to the present disclosure. The console 4 comprises a central processing unit (CPU) 41, a read only memory (ROM) 42, a random access memory (RAM) 43, and a storage 44. In addition, the console 4 may further comprise an input unit 45 and a display unit 46.

The CPU 41 which is an example of a processor is a central processing unit and executes various programs or controls each unit. That is, the CPU 41 reads out a program from the ROM 42 or the storage 44 and executes the program using the RAM 43 as a work area. The CPU 41 controls each of the above-described configurations and performs various types of arithmetic processing according to the program recorded in the ROM 42 or the storage 44. In the present embodiment, a control program of the medical diagnostic apparatus, which includes information processing of determining an imaging range in main imaging by the X-ray CT apparatus, is stored in the ROM 42 or the storage 44. The program related to the information processing corresponds to an information processing program of the present disclosure, and a processing method by the information processing corresponds to an information processing method of the present disclosure.

The ROM 42 stores various programs and various data. The RAM 43 temporarily stores a program or data as a work area. The storage 44 is configured with a storage device such as a hard disk drive (HDD), a solid state drive (SSD), or a flash memory, and stores various programs including an operating system, and various data.

The input unit 45 includes a pointing device such as a mouse, and a keyboard, and is used to perform various inputs. The input unit 45 comprises an examination table operation unit that operates a height of the examination table 3.

The display unit 46 is, for example, a liquid crystal display and displays various types of information. The display unit 46 may adopt a touch panel type to function as the input unit 45.

In the present embodiment, the console 4 is connected to a hospital information system 5 of a hospital in which the X-ray CT apparatus is installed. The hospital information system 5 comprises a patient information management system 51 and an examination reservation system 52.

The patient information management system 51 is a database in which personal information, medical care data, examination image data, examination data, medication data, and the like of a patient who has received medical care in the hospital in the past are stored. The personal information of the patient includes personal identification information such as a name, an identification (ID), a date of birth, an age, and a gender, as well as physical information such as a height and a weight, and a past examination date. These pieces of personal information are input to the system in a case where the subject visits the hospital as a patient and receives medical care. The height and the weight of the patient are collected by the patient's self-reporting on a medical questionnaire or by actual measurement, and are input to the patient information management system 51. In addition, the medical care data, the examination image data, the examination data, and the medication data are input to the patient information management system 51 each time the patient receives medical care, and an in-hospital patient database is created.

The examination reservation system 52 creates an examination schedule for that day for each system of the medical diagnostic apparatus equipped in the hospital based on information stored or written in the patient information management system 51, and distributes the created examination schedule to each system as data. The examination schedule includes an order of patients who undergo the examination on that day in the medical diagnostic apparatus and personal identification information and physical information of each patient. The content of the examination reservation system 52 is sequentially updated according to the progress of the medical care of that day, and the updated data is transmitted to each system each time the content is updated.

A camera 7 is provided on a ceiling of an imaging room in which the medical diagnostic apparatus is provided in a state in which a region within a movable range of the examination table 3 can be imaged. The camera 7 according to the present embodiment is a depth camera that can obtain depth information indicating a depth for each pixel within an imaging angle of view by imaging. In the camera 7 according to the present embodiment, a time of flight (ToF) type sensor is applied as a sensor for obtaining the depth information, but the present disclosure is not limited thereto. For example, a form may be adopted in which the depth information is obtained by a stereo camera type.

The camera 7 is used to detect a body thickness of the subject (hereinafter, also simply referred to as a "body thickness") in a state of a supine posture on the top plate 31 of the examination table 3. In the present embodiment, the camera 7 acquires depth information (hereinafter, referred to as "initial depth information") on an upper surface of the top plate 31 of the examination table 3 in a state in which there is no subject and a position of the top plate 31 of the examination table 3 in an up-down direction is set to a position in a case of the main imaging for the subject. In the present embodiment, in a state in which the subject is in a supine posture on the top plate 31 of the examination table 3 in order to actually perform the main imaging, depth information (hereinafter, referred to as "imaging depth information") on an upper surface side of the top plate 31 is acquired by the camera 7, and the initial depth information is subtracted from the acquired imaging depth information for each corresponding pixel to acquire information (hereinafter, referred to as "body thickness information") indicating the body thickness for each pixel.

As described above, in the medical diagnostic apparatus according to the present embodiment, the depth camera is applied to acquire the body thickness information, and the body thickness information is estimated by using a depth camera image obtained by imaging with the depth camera. However, as an alternative, not forming part of the invention, a form may be adopted in which an optical camera is applied as the camera 7, and a subject image included in an optical camera image obtained by imaging with the optical camera is analyzed to estimate the body thickness information. In this case, a form in which the camera 7 is installed obliquely above or on a side of the examination table 3 and the body thickness information of the subject is acquired from the optical camera image of the subject obtained by imaging with the camera 7 is exemplified. In a case where the camera 7 is provided obliquely above the examination table 3 in this form, the body thickness indicated by the optical camera image obtained by imaging is changed according to an imaging angle with respect to the subject from the camera 7. Therefore, a form in which the camera 7 is provided on the side of the examination table 3 is preferable.

In a case where the control program of the medical diagnostic apparatus is executed, the console 4 realizes various functions by using hardware resources described above. Functional configurations realized by the console 4 will be described.

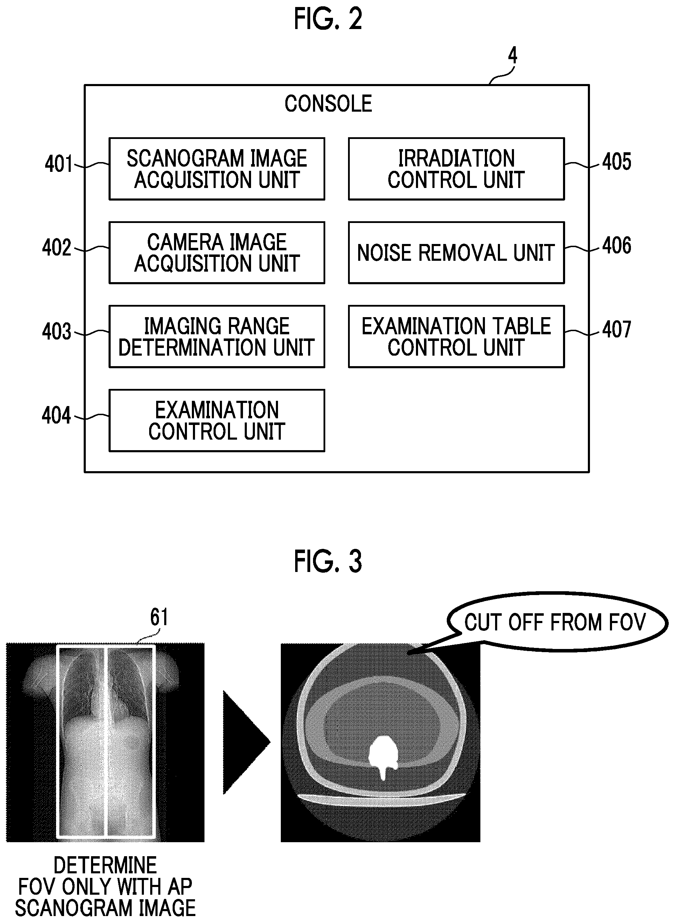

Fig. 2 is a block diagram showing an example of the functional configurations of the console 4.

As shown in Fig. 2, the console 4 has a scanogram image acquisition unit 401, a camera image acquisition unit 402, an imaging range determination unit 403, an examination control unit 404, an irradiation control unit 405, a noise removal unit 406, and an examination table control unit 407 as the functional configurations. Each functional configuration is realized by the CPU 41 reading out and executing the control program of the medical diagnostic apparatus stored in the ROM 42 or the storage 44.

The scanogram image acquisition unit 401 executes processing of acquiring a scanogram image of the subject based on irradiation of the subject with X-rays controlled by the irradiation control unit 405 described below. The scanogram image can be obtained by detecting X-rays transmitted through the subject with the X-ray detector 15. The scanogram image acquisition unit 401 acquires a scanogram image including at least the most protruding region of an abdomen (in the present embodiment, a central portion of the abdomen) of the subject.

The camera image acquisition unit 402 acquires a camera image (in the present embodiment, the depth camera image) obtained by imaging the subject with the camera 7.

Incidentally, in a case where the subject is obese and a protrusion amount of the abdomen from a front side of a body is relatively large, a front-back width is often greater than a lateral width for the subject. In this case, in the technique of the related art in which the imaging range (hereinafter, also referred to as a field of view (FOV)) in the main imaging is determined on the basis of only the scanogram image obtained by imaging from the AP direction, a part of the abdomen of the subject may be cut off (chipped) in an axial image obtained by the main imaging.

Fig. 3 is a diagram for describing a problem of the related art, and is a diagram showing an example of an FOV 61 of the main imaging and the axial image obtained by the related art. As shown in Fig. 3, in this case, a part of the abdomen of the subject is cut off in the axial image.

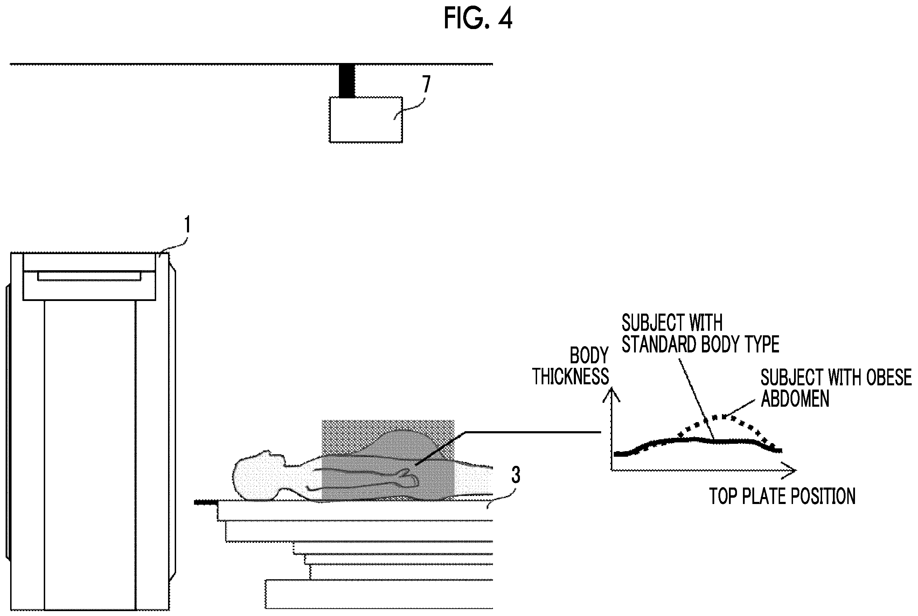

That is, as shown in Fig. 4 as an example, the body thickness of an obese subject in a state of a supine posture is significantly different in the position of the abdomen from that of the subject with a standard body type. Fig. 4 is a diagram for describing a problem of the related art, and is a side view showing an example of the medical image capturing apparatus and a graph showing a relationship between the position on the top plate 31 of the examination table 3 and the body thickness.

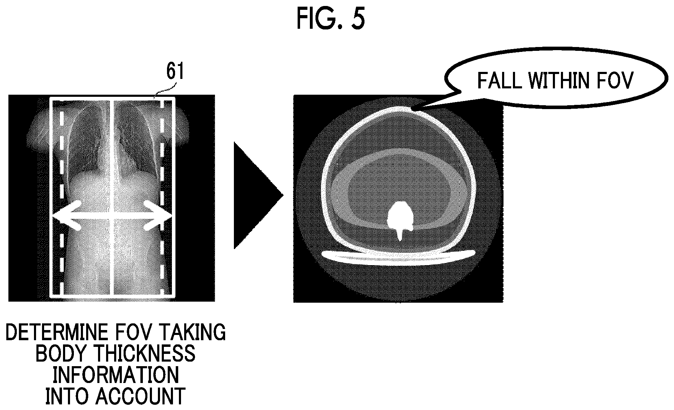

Therefore, as shown in Fig. 5 as an example, the imaging range determination unit 403 according to the present embodiment determines the FOV 61 in the main imaging using the body thickness information indicating the body thickness of the subject obtained from the camera image and the scanogram image. As a result, it is possible to avoid cutting off the abdomen of the subject in the axial image. Fig. 5 is a diagram for describing the disclosed technology, and is a diagram showing an example of the FOV 61 of the main imaging and the axial image obtained by the disclosed technology.

The examination control unit 404 controls an examination sequence of the subject by the medical diagnostic apparatus.

The irradiation control unit 405 controls an irradiation amount of the X-rays from the X-ray tube device 13 to the subject. For example, a shoulder requires a large dose. However, a lung is filled with air and thus has a small attenuation, so a small dose may be sufficient, and a liver has a large attenuation and thus requires a dose. The irradiation control unit 405 controls the irradiation amount of the X-rays to the subject according to a site of the subject. A required image quality level is different between the scanogram image and an image (image of main imaging) obtained by the examination, and the image of main imaging is required to have a higher image quality than the scanogram image. Therefore, the irradiation control unit 405 may obtain the irradiation amount of the X-rays to the subject by multiplying dose data in a case of the scanogram image by a coefficient or converting the dose data using a table. The irradiation control unit 405 can obtain an examination image of the subject having a noise amount desired by an operator by controlling the irradiation amount of the X-rays to the subject using the dose data of the X-rays in a case where the scanogram image is generated.

In a case where the noise amount of the examination image obtained by the irradiation of the subject with the X-rays exceeds an allowable amount of the desired noise amount, the noise removal unit 406 performs noise removal (denoising) processing on the examination image. For example, in a case where a dose modulation based on the dose data of the subject associated with a past scanogram image does not result in a desired image quality index (for example, an image noise) due to an increase in weight of the subject and the image noise is increased, the noise removal unit 406 increases an intensity level of the denoising processing to suppress the image noise and automatically performs reconstruction to achieve a desired image quality index that is substantially the same as that in the past examination.

The examination table control unit 407 controls the height of the examination table 3. The examination table control unit 407 may control the height of the examination table 3 based on an operation of a technician, or may control the height of the examination table 3 based on data of the most recent examination of the subject.

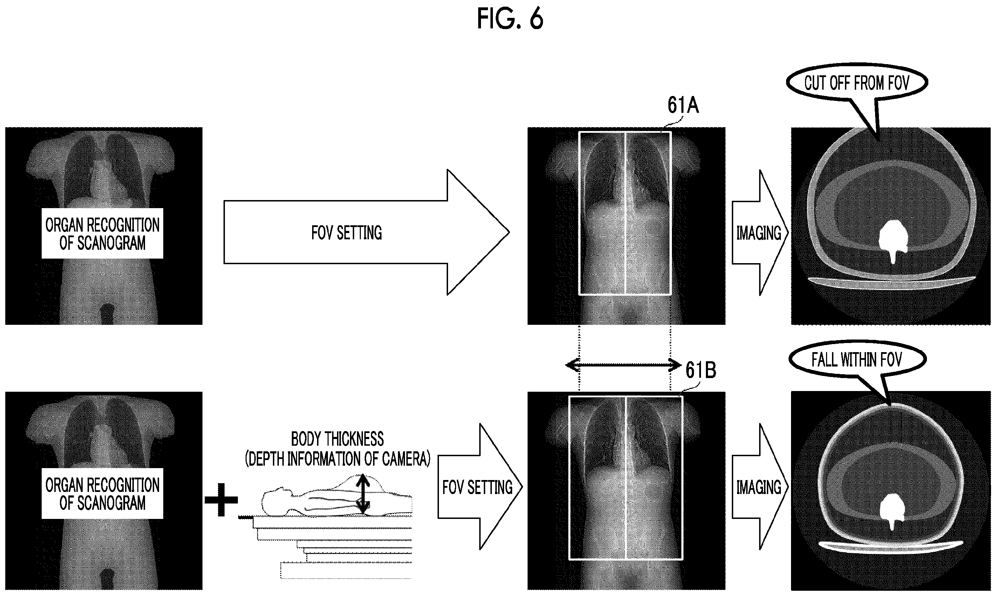

Next, processing of determining the FOV in the main imaging, which is executed in the console 4 according to the present embodiment, will be described in more detail with reference to Fig. 6. Fig. 6 is a diagram for describing the first embodiment of the disclosed technology, in which an upper diagram is a diagram showing an example of an FOV 61A of the main imaging and an axial image obtained in the same manner as the related art by the disclosed technology, and a lower diagram is a diagram showing an example of an FOV 61B of the main imaging and an axial image obtained by the disclosed technology.

First, as shown in the upper diagram of Fig. 6 as an example, the console 4 estimates the FOV (hereinafter, referred to as a "first FOV") 61A using the scanogram image in the same manner as in the related art. In this case, the estimation of the first FOV 61A may be performed, for example, by recognizing a feature point of an organ from the scanogram image and setting a region of interest (ROI) surrounding the feature point as the first FOV 61A, or by estimating the first FOV 61A using a model that sets organ recognition or an ROI using machine learning.

On the other hand, as shown in the lower diagram of Fig. 6 as an example, the console 4 estimates the body thickness of the subject as described above by using the depth information indicated by the depth camera image acquired from the camera 7. In this case, the initial depth information may be registered in advance for each height of the upper surface of the top plate 31 of the examination table 3 that can be applied to the medical diagnostic apparatus, and the initial depth information corresponding to the height of the upper surface of the top plate 31 in the main imaging may be selectively applied.

Next, the console 4 estimates the FOV (hereinafter, referred to as a "second FOV") 61B using the estimated body thickness such that the maximum value of the body thickness is a size in a lateral direction. In this case, the console 4 applies the same size as the first FOV 61A, as the size of the second FOV 61B in a longitudinal direction. However, the present disclosure is not limited to this form, and a form may be adopted in which the sizes of the first FOV 61A and the second FOV 61B in the longitudinal direction are different from each other.

Then, the console 4 compares the first FOV 61A and the second FOV 61B, and determines the FOV having a larger size in a lateral direction as a final FOV.

As described above, in the present embodiment, both the first FOV 61A and the second FOV 61B are estimated, and the FOV having a larger size in a lateral direction is applied. The reason for this is as follows.

That is, since the axial image is used for diagnosis of the subject, it is desired to have as high definition as possible, but it is desired to avoid a situation in which the abdomen of the subject is cut off. Therefore, in the console 4 according to the present embodiment, the first FOV 61A is adopted for a subject with a standard body type to obtain a higher-definition axial image, and the second FOV 61B is adopted for an obese subject to obtain an axial image in which the abdomen is not cut off.

Therefore, in a case where it is known in advance whether or not the subject is obese, the second FOV 61B may be estimated without estimating the first FOV 61A, and the second FOV 61B may be determined as the final FOV.

Next, an operation of the console 4 will be described.

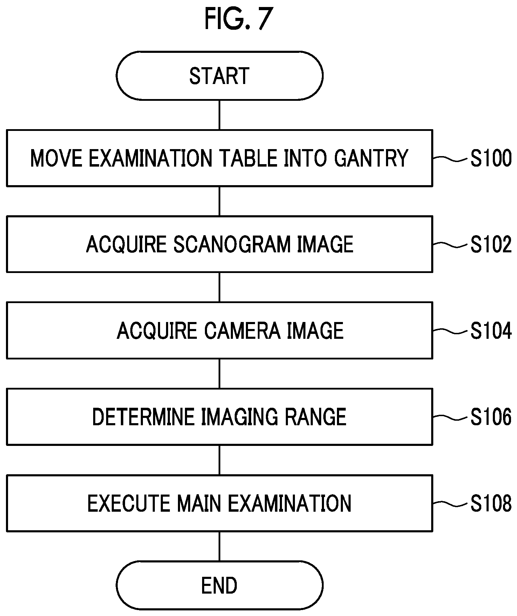

Fig. 7 is a flowchart showing a flow of control of the medical diagnostic apparatus by the console 4. The CPU 41 reads out the control program of the medical diagnostic apparatus from the ROM 42 or the storage 44, and loads and executes the control program in the RAM 43, thereby performing control processing of the medical diagnostic apparatus. The flowchart shown in Fig. 7 is executed while the subject receives an examination with the medical diagnostic apparatus.

In step S100, the CPU 41 moves the examination table 3 into the gantry 11.

Subsequent to step S100, in step S102, the CPU 41 irradiates the subject with the X-rays to acquire the scanogram image of the subject.

Subsequent to step S102, in step S104, the CPU 41 acquires the depth camera image from the camera 7 as described above.

Subsequent to step S104, in step S106, the CPU 41 determines the FOV using the acquired scanogram image and depth camera image as described above.

Subsequent to step S106, in step S108, the CPU 41 executes a main examination of the subject by applying the determined FOV.

As described above, according to the embodiment of the present disclosure, the scanogram image obtained by imaging the subject and the camera image obtained by imaging the subject with the depth camera are acquired, and the body thickness information indicating the body thickness of the subject obtained from the camera image and the scanogram image are used to determine the imaging range in the main imaging. Therefore, it is possible to suppress occurrence of chipping in the axial image obtained by the main imaging.

### Second Embodiment

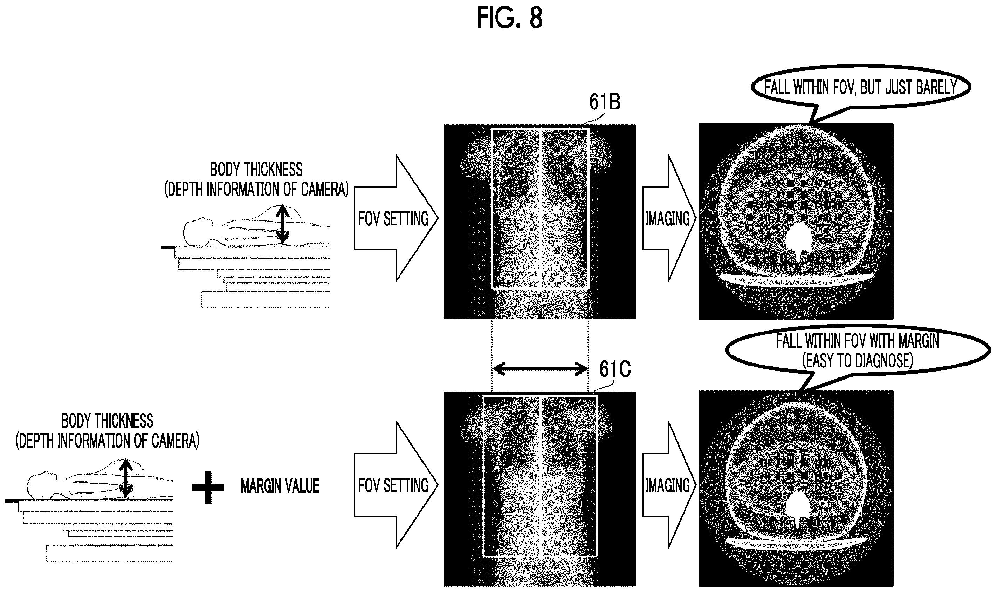

Fig. 8 is a diagram for describing a second embodiment of the disclosed technology, in which an upper diagram is a diagram showing an example of the second FOV 61B of the main imaging and an axial image obtained by the disclosed technology, and a lower diagram is a diagram showing an example of a third FOV 61C of the main imaging and an axial image obtained by adding a margin by the disclosed technology.

As shown in the upper diagram of Fig. 8 as an example, in a case where the size of the body thickness is directly applied to the FOV in a case where the FOV is estimated based on the body thickness as in the first embodiment, the subject image may be adjacent to an end part of the axial image on the captured axial image, which may result in an image that is difficult to diagnose.

Therefore, in the imaging range determination unit 403 of the console 4 according to the second embodiment, as shown in the lower diagram of Fig. 8 as an example, the FOV (in the example shown in Fig. 8, the third FOV 61C) is determined by including a predetermined margin in the body thickness information indicating the body thickness. As a result, the body of the subject can fall within the FOV with a margin, and thus the captured axial image can be made into an image that is easy to diagnose.

In this case, for example, the margin may be set to any fixed value by the user, may be calculated by multiplying the estimated body thickness by a certain ratio, or may be calculated using a model using machine learning. In addition, the margin may be changed for each unit that is desired to be used selectively in clinical practice, such as information (gender, height, weight, and the like) of the subject, an examination or diagnosis target, and the like. For example, in an examination in which it is easier to make a diagnosis in a case where the ROI is expanded, a relatively large margin is set.

The operation of the console 4 according to the present embodiment is the same as that of the first embodiment except that the margin is added to the determination of the FOV in step S106. Therefore, the description thereof will be omitted here.

### Third Embodiment

Incidentally, in a case of planning imaging using a medical diagnostic apparatus, in general, the final FOV is determined by operating a scan planning line indicating the FOV on the captured scanogram image.

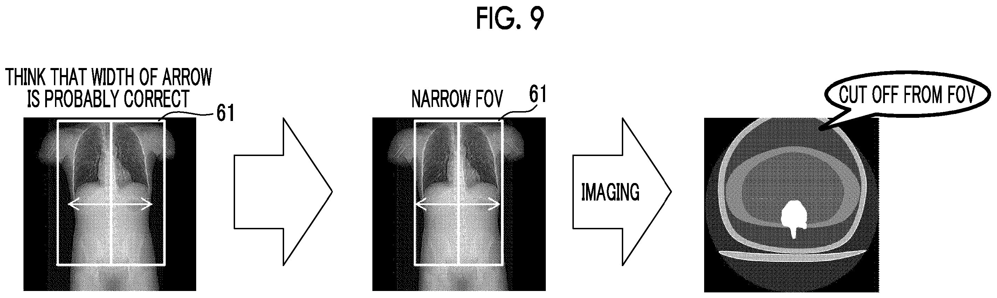

In this case, in a case where the FOV is determined according to the body thickness as in each of the above embodiments, as shown in Fig. 9 as an example, a width of the FOV 61 of the scan planning line appears not to match a lateral width of the subject image shown on the scanogram image. Therefore, there is a possibility that the user narrows the FOV 61. Fig. 9 is a diagram for describing a problem of the disclosed technology, and is a diagram showing an example of a defect in the axial image caused by the narrowing the FOV 61 due to misunderstanding by the user.

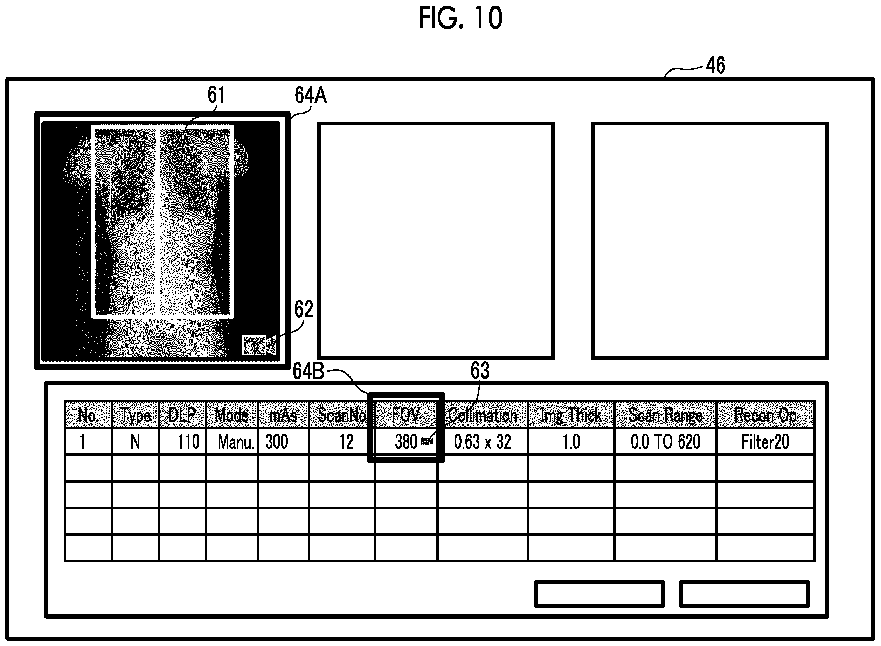

Therefore, in the console 4 according to the third embodiment, as shown in Fig. 10 as an example, in a case where the FOV estimated based on the body thickness is set as the FOV61, information to that effect is displayed on a GUI to make a notification. As a result, the user can ascertain whether or not the FOV is set based on the body thickness, and human error can be prevented. Fig. 10 is a diagram showing an example of a state of a notification on a GUI screen displayed in a case of planning the main imaging according to the third embodiment of the disclosed technology.

As shown in Fig. 10, in this form, the display on the GUI screen may be, for example, such that an icon 62 is displayed on the scanogram image, an icon 63 is displayed in a portion where the FOV or the like is set by a numerical value, or colors, line widths, or line types of lines 64A and 64B are changed. In the example shown in Fig. 10, a case where icons indicating cameras are applied as the icon 62 and the icon 63 is illustrated.

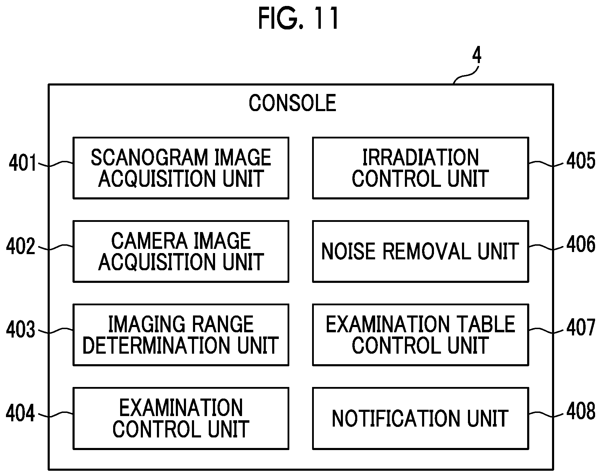

Fig. 11 is a block diagram showing an example of a functional configuration of the console 4 according to the present embodiment. As shown in Fig. 11, the console 4 according to the present embodiment is different from the console 4 according to each of the above embodiments in that a notification unit 408 is added. The notification unit 408 according to the present embodiment notifies that the FOV is determined using the body thickness information.

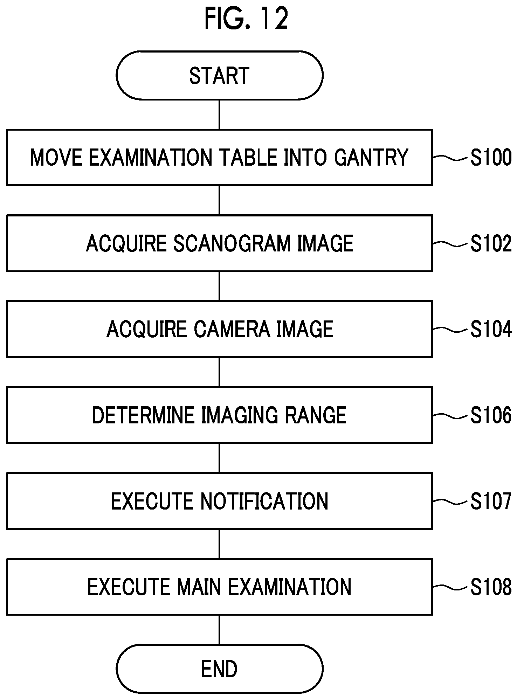

Fig. 12 is a flowchart showing a flow of control of the medical diagnostic apparatus by the console 4 according to the present embodiment, in which the same processing steps as those shown in Fig. 7 are assigned the same step numbers as those in Fig. 7, and the description thereof will be omitted.

In step S107, the CPU 41 performs control to give a notification that the FOV determined by the processing of step S106 is determined using the body thickness information. With this control, in a screen displayed on the display unit 46 or the like thereafter, as shown in Fig. 10 as an example, the icons 62 and 63, and the like are displayed, or colors, line widths, line types, and the like of the lines 64A and 64B are changed.

### Fourth Embodiment

In each of the above embodiments, the body thickness of the subject is estimated using the depth information obtained by the camera 7, and the FOV is estimated based on the body thickness. However, in a case where the body thickness of the subject is not constant due to respiration or the like, there is a possibility that the FOV may be insufficient depending on a timing of acquiring the depth information or the like.

Therefore, in the imaging range determination unit 403 of the console 4 according to the fourth embodiment, in addition to the depth information, respiratory information indicating a respiratory cycle of the subject is also acquired, and the FOV is dynamically adjusted using the respiratory information to set the FOV following a body motion caused by the respiration of the subject. That is, in the imaging range determination unit 403 according to the present embodiment, the body motion of the subject is reflected in the FOV.

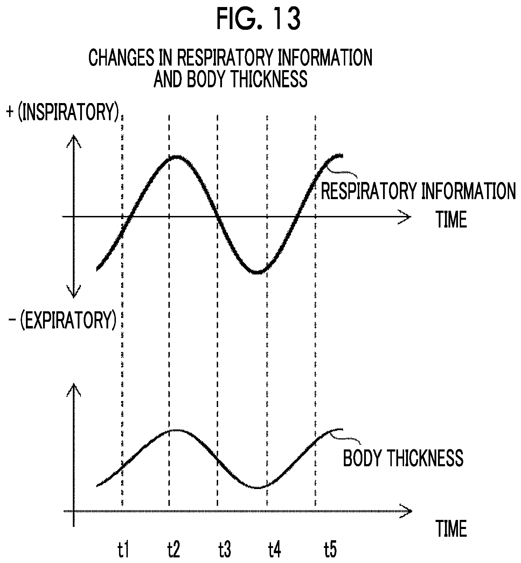

For example, as shown in Fig. 13 as an example, the respiratory information is acquired before the depth information of the camera 7 is acquired, and the respiratory information is also acquired at a timing at which the depth information is acquired from the camera 7. It is estimated which timing of the respiratory cycle of the subject is the respiration of the subject at the timing at which the depth information of the camera 7 is acquired, and the body thickness is estimated by combining a change in body thickness according to the respiratory cycle, the depth information from the camera 7 to a surface of the subject, and the depth information from the camera 7 to a surface of the top plate 31 of the examination table 3. Fig. 13 is a graph showing an example of changes in respiratory information and body thickness over time according to the fourth embodiment of the disclosed technology.

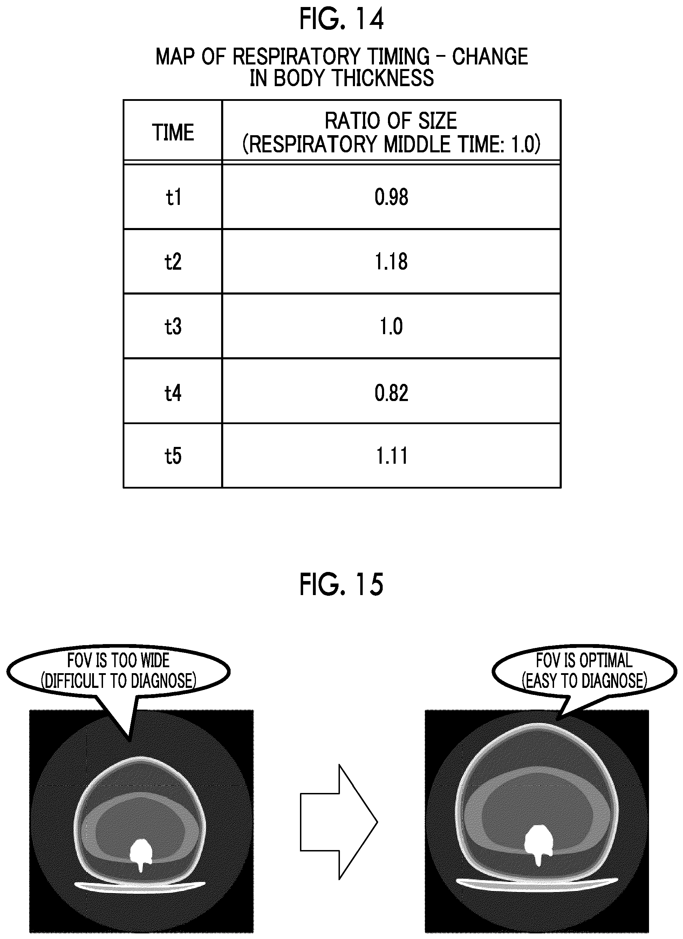

For example, as shown in Fig. 14 as an example, a ratio of a change in body thickness of a general subject is mapped for each respiratory cycle, and a maximum value of the body thickness of the subject is estimated using the ratio in a case of acquiring the depth information from the camera 7 based on the mapping, and the FOV is set according to the maximum value. Fig. 14 is a map showing an example of a relationship between a respiratory timing and a ratio of a change in body thickness according to the fourth embodiment of the disclosed technology, and in the example shown in Fig. 14, the ratio at a central timing in the respiratory cycle is set to 1.0.

In addition, the operation of the console 4 according to the present embodiment is different from that of the first embodiment only in the operation of the imaging range determination unit 403, and other operations are the same as those of the first embodiment so that the description thereof will be omitted here.

### Fifth Embodiment

In each of the above embodiments, the FOV determined by estimating the body thickness of the subject using the depth information obtained by the camera 7 may have a range that is too wide compared to an ideal FOV due to an influence of an acquisition accuracy of the depth information of the camera 7, as shown in a left diagram of Fig. 15 as an example.

Therefore, in the imaging range determination unit 403 of the console 4 according to the fifth embodiment, as shown in a right diagram of Fig. 15 as an example, a region in which the subject is shown is detected with respect to the reconstructed axial image, and the FOV is optimized according to the region. Then, the examination control unit 404 according to the present embodiment automatically reconstructs the axial image using the optimized FOV. As a result, it is possible to obtain the axial image with the optimal FOV without increasing the time and effort of the user. Fig. 15 is a diagram for describing the fifth embodiment of the disclosed technology, in which the left diagram is a diagram showing an example of a defect in the axial image by the disclosed technology, and the right diagram is a diagram showing an example of an axial image in which the defect is eliminated by the reconstruction by the disclosed technology.

The processing of detecting the region in which the subject is shown with respect to the axial image is performed by, for example, creating the axial image in real time during imaging, and detecting the region in which the subject is shown in real time immediately after the creation. Then, in a case where the imaging is completed, the optimal FOV is determined based on detection results for all the created axial images, and the axial image is reconstructed with the determined FOV.

Here, a method of determining the optimal FOV may be, for example, to determine a region encompassing the largest region among the detection results for all the axial images as the FOV, to determine the optimal FOV by multiplying an average value of the detection results for all the axial images by a constant value, or to determine the optimal FOV using a machine-learned model that outputs the optimal FOV for the subject on the axial image.

In addition, the operation of the console 4 according to the present embodiment is different from that of the first embodiment only in the operations of the imaging range determination unit 403 and the examination control unit 404, and other operations are the same as those of the first embodiment so that the description thereof will be omitted here.

Furthermore, as the model using machine learning exemplified in some of the above embodiments, in addition to a model using artificial intelligence (AI) using a convolutional neural network (CNN), for example, other machine learning models such as AI other than CNN, such as a recurrent neural network (RNN), can be applied.

Although the embodiments of the present disclosure have been described in detail with reference to the accompanying drawings, the technical scope of the present disclosure is not limited to such examples. It is apparent that those having ordinary knowledge in the technical field of the present disclosure can conceive various change examples or modification examples within the technical idea described in the claims, and it is also understood that these change examples or modification examples belong to the technical scope of the present disclosure.

In addition, the effects described in the above embodiments are merely explanatory or exemplary, and the present disclosure is not limited to the effects described in the above embodiments. That is, the technology according to the present disclosure can achieve other effects that are obvious to those having ordinary knowledge in the technical field of the present disclosure in addition to the effects described in the above embodiments, or instead of the effects described in the above embodiments.

For example, in each of the above embodiments, the information processing apparatus according to the present disclosure is applied to the X-ray CT apparatus, but the present disclosure is not limited thereto. In a case of an imaging apparatus that sets an imaging range using a scanogram image, the information processing apparatus according to the present disclosure may be applied to an MRI apparatus or the like.

In addition, in each of the above embodiments, for example, as a hardware structure of processing units that execute various types of processing, such as the scanogram image acquisition unit 401, the camera image acquisition unit 402, the imaging range determination unit 403, the examination control unit 404, the irradiation control unit 405, the noise removal unit 406, the examination table control unit 407, and the notification unit 408, various processors shown below can be used. As described above, in addition to the CPU which is a general-purpose processor executing software (program) to function as various processing units, the various processors include a programmable logic device (PLD) which is a processor capable of changing a circuit configuration after manufacture such as a field programmable gate array (FPGA), a dedicated electric circuitry which is a processor having a circuit configuration exclusively designed to execute specific processing such as an application specific integrated circuit (ASIC), and the like.

One processing unit may be configured by one of the various processors, or may be configured by a combination of the same type or different types of two or more processors (for example, a combination of a plurality of FPGAs or a combination of the CPU and the FPGA). A plurality of processing units may be configured by one processor.

As an example of configuring a plurality of processing units with one processor, first, as represented by computers such as a client and a server, there is a form in which one processor is configured by combining one or more CPUs and software, and the processor functions as a plurality of processing units. Second, as represented by a system on chip (SoC) or the like, there is a form in which a processor that realizes functions of an entire system including a plurality of processing units with one integrated circuit (IC) chip is used. As described above, the various processing units are configured using one or more of the various processors as a hardware structure.

Furthermore, as the hardware structure of the various processors, more specifically, an electric circuitry in which circuit elements such as semiconductor elements are combined can be used.

In addition, in each of the above embodiments, an aspect in which the information processing program is stored (installed) in advance in the ROM 42 or the storage 44 of the console 4 has been described, but the present disclosure is not limited thereto. The information processing program may be provided in a form recorded in a recording medium such as a compact disc read only memory (CD-ROM), a digital versatile disc read only memory (DVD-ROM), and a universal serial bus (USB) memory. Further, the information processing program may be downloaded from an external device via a network.

## Claims

1. An information processing apparatus (4) for an X-ray computed tomography (CT) apparatus comprising a processor (41),
wherein the processor (41) is configured to:
acquire a scanogram image obtained by imaging a subject and camera images obtained by imaging the subject with a depth camera (7);
acquire, using the depth camera (7), first depth information on an upper surface of a top plate (31) of an examination table (3) in a state in which there is no subject and a position of the top plate (31) of the examination table (3) is set to a position used in main imaging of the subject;
acquire, using the depth camera (7), second depth information on the upper surface of the top plate (31) of the examination table (3) in a state in which the subject is in a supine posture on the examination table (3) in order to actually perform the main imaging;
generate the body thickness information by subtracting the first depth information from the second depth information for each corresponding pixel;
estimate a first field of view (61A) using the scanogram image;
estimate a second field of view (61B) using the generated body thickness information such that a maximum value of the body thickness is a size in a lateral direction; and
determine an imaging range (61) for main imaging by comparing the first field of view (61A) and the second field of view (61B) and selecting the field of view having a larger size in a lateral direction as a final imaging range (61).

2. The information processing apparatus (4) according to claim 1,
wherein the processor (41) is configured to:
determine the imaging range (61) by including a predetermined margin in the body thickness information.

3. The information processing apparatus (4) according to claim 1,
wherein the processor (41) is configured to:
notify that the imaging range (61) is determined using the body thickness information.

4. The information processing apparatus (4) according to claim 1,
wherein the processor (41) is configured to:
reflect a body motion of the subject in the imaging range (61).

5. The information processing apparatus (4) according to claim 1,
wherein the processor (41) is configured to:
adjust the determined imaging range (61) using an axial image of the subject to reconstruct the axial image in the main imaging.

6. A medical image capturing apparatus (1, 3, and 4) comprising:
the information processing apparatus (4) according to claim 1, and
a radiography apparatus (1) controlled by the information processing apparatus (4).

7. An information processing method for an X-ray computed tomography (CT) apparatus comprising:
via a processor (41),
acquiring a scanogram image obtained by imaging a subject and camera images obtained by imaging the subject with a depth camera (7);
acquiring, using the depth camera (7), first depth information on an upper surface of a top plate (31) of an examination table (3) in a state in which there is no subject and a position of the top plate (31) of the examination table (3) is set to a position used in main imaging of the subject;
acquiring, using the depth camera (7), second depth information on the upper surface of the top plate (31) of the examination table (3) in a state in which the subject is in a supine posture on the examination table (3) in order to actually perform the main imaging;
generating the body thickness information by subtracting the first depth information from the second depth information for each corresponding pixel;
estimating a first field of view (61A) using the scanogram image;
estimating a second field of view (61B) using the generated body thickness information such that a maximum value of the body thickness is a size in a lateral direction; and
determining an imaging range (61) for main imaging by comparing the first field of view (61A) and the second field of view (61B) and selecting the field of view having a larger size in a lateral direction as a final imaging range (61).

8. A storage medium (42, 44) storing an information processing program for an X-ray computed tomography (CT) apparatus, executable by a computer (4) to execute a process comprising:
acquiring a scanogram image obtained by imaging a subject and camera images obtained by imaging the subject with a depth camera (7); and
acquiring, using the depth camera (7), first depth information on an upper surface of a top plate (31) of an examination table (3) in a state in which there is no subject and a position of the top plate (31) of the examination table (3) is set to a position used in main imaging of the subject;
acquiring, using the depth camera (7), second depth information on the upper surface of the top plate (31) of the examination table (3) in a state in which the subject is in a supine posture on the examination table (3); and
generating the body thickness information by subtracting the first depth information from the second depth information for each corresponding pixel;
estimating a first field of view (61A) using the scanogram image;
estimating a second field of view (61B) using the generated body thickness information such that a maximum value of the body thickness is a size in a lateral direction; and
determining an imaging range (61) for main imaging by comparing the first field of view (61A) and the second field of view (61B) and selecting the field of view having a larger size in a lateral direction as a final imaging range (61).

## Patentansprüche

1. Informationsverarbeitungsvorrichtung (4) für eine Röntgen-Computertomographie(CT)-Vorrichtung, umfassend einen Prozessor (41),
wobei der Prozessor (41) so konfiguriert ist, dass er:
ein Scanogramm-Bild, das durch Abbilden einer Untersuchungsperson erhalten wird, und Kamerabilder, die durch Abbilden der Untersuchungsperson mit einer Tiefenkamera (7) erhalten werden, erfasst;
unter Verwendung der Tiefenkamera (7) erste Tiefeninformationen über eine obere Oberfläche einer Deckplatte (31) eines Untersuchungstisches (3) in einem Zustand, in dem es keine Untersuchungsperson gibt und eine Position der Deckplatte (31) des Untersuchungstisches (3) auf eine Position eingestellt ist, die bei Hauptbildgebung der Untersuchungsperson verwendet wird, erfasst;
unter Verwendung der Tiefenkamera (7) zweite Tiefeninformationen über die obere Oberfläche der Deckplatte (31) des Untersuchungstisches (3) in einem Zustand, in dem sich die Untersuchungsperson in einer Rückenlage auf dem Untersuchungstisch (3) befindet, um die Hauptbildgebung tatsächlich durchzuführen, erfasst;
die Körperdickeninformationen durch Subtrahieren der ersten Tiefeninformationen von den zweiten Tiefeninformationen für jedes entsprechende Pixel erzeugt;
ein erstes Sichtfeld (61A) unter Verwendung des Scanogramm-Bildes schätzt;
ein zweites Sichtfeld (61B) unter Verwendung der erzeugten Körperdickeninformationen so schätzt, dass ein Maximalwert der Körperdicke eine Größe in einer Querrichtung ist; und
einen Bildgebungsbereich (61) für Hauptbildgebung durch Vergleichen des ersten Sichtfelds (61A) und des zweiten Sichtfelds (61B) und Auswählen des Sichtfelds mit einer größeren Größe in einer Querrichtung als einen endgültigen Bildgebungsbereich (61) bestimmt.

2. Informationsverarbeitungsvorrichtung (4) nach Anspruch 1,
wobei der Prozessor (41) so konfiguriert ist, dass er:
den Bildgebungsbereich (61) durch Einschließen eines vorbestimmten Randes in die Körperdickeninformationen bestimmt.

3. Informationsverarbeitungsvorrichtung (4) nach Anspruch 1,
wobei der Prozessor (41) so konfiguriert ist, dass er:
benachrichtigt, dass der Bildgebungsbereich (61) unter Verwendung der Körperdickeninformationen bestimmt wird.

4. Informationsverarbeitungsvorrichtung (4) nach Anspruch 1,
wobei der Prozessor (41) so konfiguriert ist, dass er:
eine Körperbewegung der Untersuchungsperson in dem Bildgebungsbereich (61) widerspiegelt.

5. Informationsverarbeitungsvorrichtung (4) nach Anspruch 1,
wobei der Prozessor (41) so konfiguriert ist, dass er:
den bestimmten Bildgebungsbereich (61) unter Verwendung eines axialen Bildes der Untersuchungsperson anpasst, um das axiale Bild bei der Hauptbildgebung zu rekonstruieren.

6. Medizinische Bildaufnahmevorrichtung (1, 3 und 4), umfassend:
die Informationsverarbeitungsvorrichtung (4) nach Anspruch 1, und
eine Röntgenvorrichtung (1), die von der Informationsverarbeitungsvorrichtung (4) gesteuert wird.

7. Informationsverarbeitungsverfahren für eine Röntgen-Computertomographie(CT)-Vorrichtung, umfassend:
via einen Prozessor (41),
Erfassen eines Scanogramm-Bildes, das durch Abbilden einer Untersuchungsperson erhalten wird, und von Kamerabildern, die durch Abbilden der Untersuchungsperson mit einer Tiefenkamera (7) erhalten werden;
Erfassen, unter Verwendung der Tiefenkamera (7), von ersten Tiefeninformationen über eine obere Oberfläche einer Deckplatte (31) eines Untersuchungstisches (3) in einem Zustand, in dem es keine Untersuchungsperson gibt und eine Position der Deckplatte (31) des Untersuchungstisches (3) auf eine Position eingestellt ist, die bei Hauptbildgebung der Untersuchungsperson verwendet wird;
Erfassen, unter Verwendung der Tiefenkamera (7), von zweiten Tiefeninformationen über die obere Oberfläche der Deckplatte (31) des Untersuchungstisches (3) in einem Zustand, in dem sich die Untersuchungsperson in einer Rückenlage auf dem Untersuchungstisch (3) befindet, um die Hauptbildgebung tatsächlich durchzuführen;
Erzeugen der Körperdickeninformationen durch Subtrahieren der ersten Tiefeninformationen von den zweiten Tiefeninformationen für jedes entsprechende Pixel;
Schätzen eines ersten Sichtfelds (61A) unter Verwendung des Scanogramm-Bildes;
Schätzen eines zweiten Sichtfelds (61B) unter Verwendung der erzeugten Körperdickeninformationen derart, dass ein Maximalwert der Körperdicke eine Größe in einer Querrichtung ist; und
Bestimmen eines Bildgebungsbereichs (61) für Hauptbildgebung durch Vergleichen des ersten Sichtfelds (61A) und des zweiten Sichtfelds (61B) und Auswählen des Sichtfelds mit einer größeren Größe in einer Querrichtung als einen endgültigen Bildgebungsbereich (61).

8. Speichermedium (42, 44), das ein Informationsverarbeitungsprogramm für eine Röntgen-Computertomographie(CT)-Vorrichtung speichert, das von einem Computer (4) ausführbar ist, um einen Prozess auszuführen, der umfasst:
Erfassen eines Scanogramm-Bildes, das durch Abbilden einer Untersuchungsperson erhalten wird, und von Kamerabildern, die durch Abbilden der Untersuchungsperson mit einer Tiefenkamera (7) erhalten werden; und
Erfassen, unter Verwendung der Tiefenkamera (7), von ersten Tiefeninformationen über eine obere Oberfläche einer Deckplatte (31) eines Untersuchungstisches (3) in einem Zustand, in dem es keine Untersuchungsperson gibt und eine Position der Deckplatte (31) des Untersuchungstisches (3) auf eine Position eingestellt ist, die bei Hauptbildgebung der Untersuchungsperson verwendet wird;
Erfassen, unter Verwendung der Tiefenkamera (7), von zweiten Tiefeninformationen über die obere Oberfläche der Deckplatte (31) des Untersuchungstisches (3) in einem Zustand, in dem sich die Untersuchungsperson in einer Rückenlage auf dem Untersuchungstisch (3) befindet; und
Erzeugen der Körperdickeninformationen durch Subtrahieren der ersten Tiefeninformationen von den zweiten Tiefeninformationen für jedes entsprechende Pixel;
Schätzen eines ersten Sichtfelds (61A) unter Verwendung des Scanogramm-Bildes;
Schätzen eines zweiten Sichtfelds (61B) unter Verwendung der erzeugten Körperdickeninformationen derart, dass ein Maximalwert der Körperdicke eine Größe in einer Querrichtung ist; und
Bestimmen eines Bildgebungsbereichs (61) für Hauptbildgebung durch Vergleichen des ersten Sichtfelds (61A) und des zweiten Sichtfelds (61B) und Auswählen des Sichtfelds mit einer größeren Größe in einer Querrichtung als einen endgültigen Bildgebungsbereich (61).

## Revendications

1. Appareil de traitement d'informations (4) pour un appareil de tomodensitométrie (computed tomography, CT) à rayons X comprenant un processeur (41),
dans lequel le processeur (41) est configuré pour :
acquérir une image de scanogramme obtenue en imageant un sujet et des images de caméra obtenues en imageant le sujet avec une caméra de profondeur (7) ;
acquérir, en utilisant la caméra de profondeur (7), des premières informations de profondeur sur une surface supérieure d'une plaque supérieure (31) d'une table d'examen (3) dans un état dans lequel il n'y a pas de sujet et une position de la plaque supérieure (31) de la table d'examen (3) est réglée sur une position utilisée dans l'imagerie principale du sujet ;
acquérir, en utilisant la caméra de profondeur (7), des deuxièmes informations de profondeur sur la surface supérieure de la plaque supérieure (31) de la table d'examen (3) dans un état dans lequel le sujet est en décubitus dorsal sur la table d'examen (3) afin de réaliser effectivement l'imagerie principale ;
générer les informations d'épaisseur corporelle en soustrayant les premières informations de profondeur des deuxièmes informations de profondeur pour chaque pixel correspondant ;
estimer un premier champ de vision (61A) en utilisant l'image de scanogramme ;
estimer un deuxième champ de vision (61B) en utilisant les informations d'épaisseur corporelle générées de sorte qu'une valeur maximale de l'épaisseur corporelle soit une taille dans une direction latérale ; et
déterminer une plage d'imagerie (61) pour l'imagerie principale en comparant le premier champ de vision (61A) et le deuxième champ de vision (61B) et en sélectionnant le champ de vision ayant une taille plus grande dans une direction latérale comme plage d'imagerie finale (61).

2. Appareil de traitement d'informations (4) selon la revendication 1,
dans lequel le processeur (41) est configuré pour :
déterminer la plage d'imagerie (61) en incluant une marge prédéterminée dans les informations d'épaisseur corporelle.

3. Appareil de traitement d'informations (4) selon la revendication 1,
dans lequel le processeur (41) est configuré pour :
notifier que la plage d'imagerie (61) est déterminée en utilisant les informations d'épaisseur corporelle.

4. Appareil de traitement d'informations (4) selon la revendication 1,
dans lequel le processeur (41) est configuré pour :
refléter un mouvement corporel du sujet dans la plage d'imagerie (61).

5. Appareil de traitement d'informations (4) selon la revendication 1,
dans lequel le processeur (41) est configuré pour :
ajuster la plage d'imagerie déterminée (61) en utilisant une image axiale du sujet pour reconstruire l'image axiale dans l'imagerie principale.

6. Appareil de capture d'images médicales (1, 3 et 4) comprenant :
l'appareil de traitement d'informations (4) selon la revendication 1, et
un appareil de radiographie (1) contrôlé par l'appareil de traitement d'informations (4).

7. Procédé de traitement d'informations pour un appareil de tomodensitométrie (CT) à rayons X comprenant :
via un processeur (41),
acquérir une image de scanogramme obtenue en imageant un sujet et des images de caméra obtenues en imageant le sujet avec une caméra de profondeur (7) ;
acquérir, en utilisant la caméra de profondeur (7), des premières informations de profondeur sur une surface supérieure d'une plaque supérieure (31) d'une table d'examen (3) dans un état dans lequel il n'y a pas de sujet et une position de la plaque supérieure (31) de la table d'examen (3) est réglée sur une position utilisée dans l'imagerie principale du sujet ;
acquérir, en utilisant la caméra de profondeur (7), des deuxièmes informations de profondeur sur la surface supérieure de la plaque supérieure (31) de la table d'examen (3) dans un état dans lequel le sujet est en décubitus dorsal sur la table d'examen (3) afin de réaliser effectivement l'imagerie principale ;
générer les informations d'épaisseur corporelle en soustrayant les premières informations de profondeur des deuxièmes informations de profondeur pour chaque pixel correspondant ;
estimer un premier champ de vision (61A) en utilisant l'image de scanogramme ;
estimer un deuxième champ de vision (61B) en utilisant les informations d'épaisseur corporelle générées de sorte qu'une valeur maximale de l'épaisseur corporelle soit une taille dans une direction latérale ; et
déterminer une plage d'imagerie (61) pour l'imagerie principale en comparant le premier champ de vision (61A) et le deuxième champ de vision (61B) et en sélectionnant le champ de vision ayant une taille plus grande dans une direction latérale comme plage d'imagerie finale (61).

8. Support de stockage (42, 44) stockant un programme de traitement d'informations pour un appareil de tomodensitométrie (CT) à rayons X, exécutable par un ordinateur (4) pour exécuter un processus comprenant :
acquérir une image de scanogramme obtenue en imageant un sujet et des images de caméra obtenues en imageant le sujet avec une caméra de profondeur (7) ; et
acquérir, en utilisant la caméra de profondeur (7), des premières informations de profondeur sur une surface supérieure d'une plaque supérieure (31) d'une table d'examen (3) dans un état dans lequel il n'y a pas de sujet et une position de la plaque supérieure (31) de la table d'examen (3) est réglée sur une position utilisée dans l'imagerie principale du sujet ;
acquérir, en utilisant la caméra de profondeur (7), des deuxièmes informations de profondeur sur la surface supérieure de la plaque supérieure (31) de la table d'examen (3) dans un état dans lequel le sujet est en décubitus dorsal sur la table d'examen (3) ; et
générer les informations d'épaisseur corporelle en soustrayant les premières informations de profondeur des deuxièmes informations de profondeur pour chaque pixel correspondant ;
estimer un premier champ de vision (61A) en utilisant l'image de scanogramme ;
estimer un deuxième champ de vision (61B) en utilisant les informations d'épaisseur corporelle générées de sorte qu'une valeur maximale de l'épaisseur corporelle soit une taille dans une direction latérale ; et
déterminer une plage d'imagerie (61) pour l'imagerie principale en comparant le premier champ de vision (61A) et le deuxième champ de vision (61B) et en sélectionnant le champ de vision ayant une taille plus grande dans une direction latérale comme plage d'imagerie finale (61).
